# EUROPÄISCHE PATENTSCHRIFT

(11) **EP 2 277 320 B1**
(45) Veröffentlichungstag und Bekanntmachung des Hinweises auf die Patenterteilung: **17.10.2018**
(21) Anmeldenummer: 09745280.9
(22) Anmeldetag: 14.05.2009
(51) Int. Cl.: A61B 5/053, G01R 21/133, H04Q 9/00

(54) **VERFAHREN ZUR DRAHTLOSEN DATENÜBERTRAGUNG ZWISCHEN EINEM MESSBAUSTEIN UND EINER ÜBERTRAGUNGSEINHEIT**
METHOD FOR WIRELESS DATA TRANSMISSION BETWEEN A MEASUREMENT MODULE AND A TRANSMISSION UNIT
PROCÉDÉ DE TRANSMISSION SANS FIL DE DONNÉES ENTRE UN MODULE DE MESURE ET UNE UNITÉ DE TRANSMISSION

(30) Priorität: 14.05.2008 AT 7702008
(43) Veröffentlichungstag der Anmeldung: 26.01.2011
(73) Patentinhaber: AIT Austrian Institute of Technology GmbH, 1220 Wien (AT)
(72) Erfinder: HEER, Rudolf, A-1220 Wien (AT); BRÜCKL, Hubert, A-2344 Maria Enzersdorf (AT); KROATH, Hans, A-2500 Baden (AT); WISSENWASSER, Jürgen, A-1100 Wien (AT)
(74) Vertreter: Wildhack & Jellinek
(86) Internationale Anmeldenummer: PCT/AT2009/000198
(87) Internationale Veröffentlichungsnummer: WO 2009/137858

(56) Entgegenhaltungen:
- US-A- 5 053 774
- US-A- 5 597 534
- US-A1- 2003 114 769
- US-A1- 2005 261 562
- US-B1- 6 411 212

## Beschreibung

Die Erfindung betrifft ein Verfahren gemäß dem Oberbegriff des Anspruchs 1 sowie eine Anordnung gemäß dem Oberbegriff des Anspruchs 4.

Erfindungsgemäße Verfahren und Anordnungen werden insbesondere für die online-Überwachung und Ermittlung von Messgrößen bzw. von biologischen Reaktionen, insbesondere der elektrischen Eigenschaften von Zellkulturen verwendet.

Typischerweise wird zur Bestimmung von biologischen Eigenschaften einer Probe die Impedanz dieser Probe bei einer oder mehrer vorgegebenen Frequenzen bestimmt. Durch diese Vielzahl von bei unterschiedlichen Frequenzen gemessenen Impedanzen können Rückschlüsse auf biologische Eigenschaften der Probe gezogen werden. Um die Messung einfach und kostengünstig zu gestalten, kann vorgesehen werden, dass die Messbausteine, in welchen sich die biologischen Proben befinden, und die Übertragungseinheiten, welche die Messdaten zu einer zentralen Datenverarbeitungseinheit weiterleiten, als getrennte Einheiten ausgeführt sind. Zur einfachen Übertragung zwischen der Übertragungseinheit und dem Messbaustein können insbesondere drahtlose Datenübertragungsverfahren, insbesondere RFID-Übertragungsverfahren, herangezogen werden. Bevorzugterweise kann vorgesehen werden, dass die Messbausteine als passive RFID-Komponenten realisiert sind, d.h., dass die Energieversorgung der Messbausteine über ein von der Übertragungseinheit ausgehendes elektromagnetisches Signal erfolgt, wobei im Messbaustein eine Energiezwischenspeicherung erfolgt.

US 5,597,534 A zeigt ein Beispiel einer solchen Anordnung. Ein wesentliches Problem des Standes der Technik besteht darin, dass die Messung der Impedanz der biologischen Proben sowie die Übertragung der Messdaten mittels rasch veränderlicher elektromagnetischer Felder realisiert ist. Aus diesem Grund kann es während der Aufnahme der Messdaten zu Interferenzen kommen, welche durch die Übertragung bereits zuvor gemessener Messdaten, oder durch die simultane Energieübertragung, verursacht wird.

Die Erfindung hat die Aufgabe, die genannten Probleme zu lösen und ein Verfahren bzw. eine Messanordnung zu schaffen, welche die genannten Probleme überwindet.

Die Erfindung löst dieses Problem bei einem Verfahren mit den Merkmalen des Kennzeichens des Anspruchs 1 sowie bei einer Anordnung mit den Merkmalen des Kennzeichens des Anspruchs 4.

Ein wesentlicher Vorteil des erfindungsgemäßen Verfahrens und der erfindungsgemäßen Anordnung besteht darin, dass während der Erfassung der Messgrößen durch den Messbaustein keinerlei Interferenzen zwischen dem für die Messung benötigten elektromagnetischen Signal und dem Signal für die Daten- oder Energieübertragung auftreten. Die Genauigkeit bzw. das Signal-Rauschverhältnis (SNR) der Messung wird somit stark verbessert, wobei insbesondere die Qualität der Impedanzmessung in denjenigen Frequenzbändern bzw. deren Oberwellen oder subharmonischen Wellen besondern stark verbessert wird, welche mit dem Frequenzband zusammenfallen, welches zur Datenübertragung verwendet wird. Die Erfindung verhindert somit Interferenzen zwischen im Nahefeld zwischen den für die Daten- und Energieübermittlung verwendeten Antennen und den zur Bestimmung der Messgrößen vorgesehenen Komponenten auf dem Messbaustein.

Mit den Merkmalen des Anspruches 2 können besonders genaue Aussagen über die Beschaffenheit von biologischen Proben getroffen werden.

Mit den Merkmalen des Anspruches 3 wird ein besonders einfacher Verfahrensablauf gewährleistet, welcher eine hohe Fehlertoleranz aufweist und die Stabilität des Messvorganges erhöht.

Eine Anordnung mit den Merkmalen des Anspruches 5 erlaubt auf einfache Art und Weise die Integration einer Vielzahl von Messbausteinen auf einer gemeinsamen Übertragungseinheit.
Fig. 1 zeigt einen erfindungsgemäßen Messaufbau umfassend eine Übertragungseinheit sowie zwei Messbausteine.
Fig. 2 zeigt schematisch den Aufbau eines Messbausteins.
Fig. 3 zeigt ein Timing-Diagramm, das die temporär gespeicherte Versorgungsspannung im Messbaustein sowie die von den jeweiligen Übertragungsantennen der Übertragungseinheit bzw. der Messbausteine übermittelten Signale im Zeitverlauf darstellt.
Fig. 4 zeigt schematisch die Übertragung von Energie und Information in Form elektromagnetischer Wellen

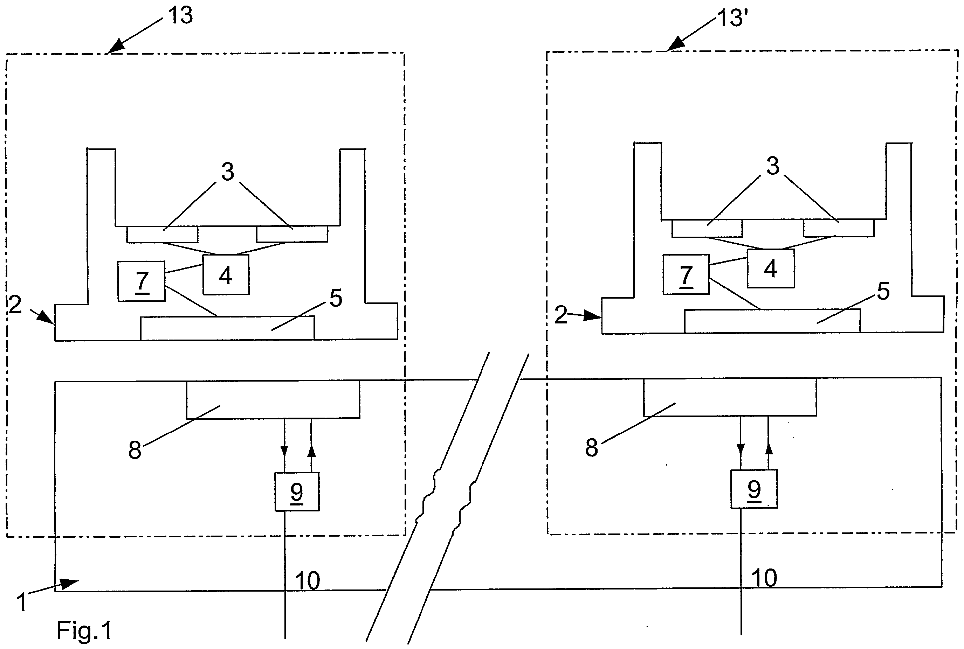

Fig. 1 zeigt eine Übertragungseinheit 1 sowie zwei mit dieser Übertragungseinheit 1 in Funkverbindung stehende Messbausteine 2. Diese Übertragungseinheit 1 umfasst dabei eine Vielzahl von ersten Antennen 8, wobei jedem Messbaustein 2 eine erste Antenne 8 zugeordnet ist, wobei sich diese erste Antenne 8 im Nahebereich des Messbausteins 2 befindet. Jeder ersten Antenne 8 ist ein Antennentreiber 9 zugeordnet, welcher eine bidirektionale Kommunikation mit der Antenne 8 ermöglicht. Ferner ist am Antennentreiber 9 ein weiterer Ausgang für eine Versorgungsleitung 10 bzw. eine gemeinsame Datenleitung 10 für eine Vielzahl von in der Übertragungseinheit 1 befindlichen ersten Antennen 8 bzw. den jeweils zugehörigen Antennentreibern 9 vorgesehen.

Oberhalb der ersten Antennen 8 sind Messbausteine 2 dargestellt, welche eine zweite Antenne 5 umfassen, die mit der jeweiligen zugeordneten ersten Antenne 8 in elektromagnetischer Wirkverbindung steht. Jede der ersten Antennen 8 ist unmittelbar unterhalb der zugeordneten zweiten Antenne 5 angeordnet, wobei zwischen den beiden einander zugeordneten Antennen 5, 8 ein Abstand von einigen Millimetern bestehen kann. Jeder Messbaustein 2 umfasst einen Probebehälter, in welchem das biologische Testmaterial eingefüllt werden kann. Die Sensoren 3 sind typischerweise am inneren Rand des jeweiligen Probenbehälters angeordnet.

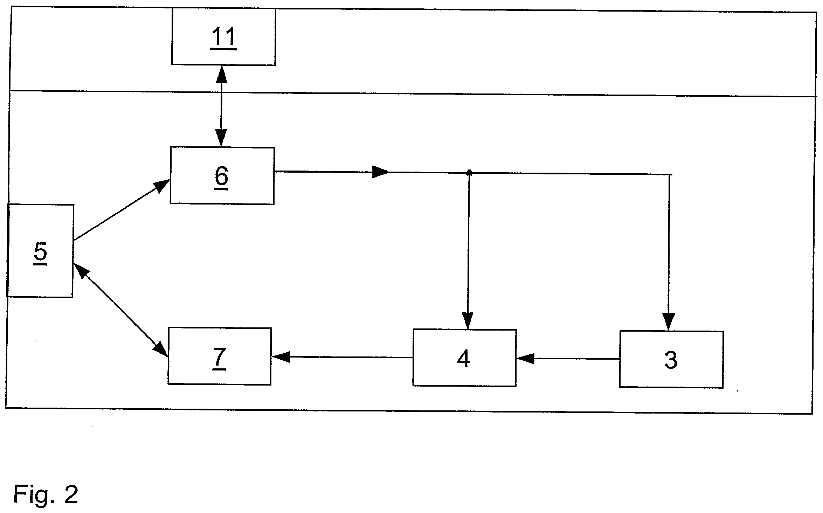

Jeder der Messbausteine 2, wie schematisch in Fig. 2 darstellt, umfasst einen oder mehrere Sensoren zur Aufnahme von mehreren physikalischen Messgrößen sowie deren Umwandlung in elektrische Signale. Diesen Sensoren sind jeweils ein oder mehrere Messverstärker 4 zur Verstärkung und gegebenenfalls zur Analog-Digital-Konversion der Signalamplitude bzw. des Signalverlaufs nachgeschaltet. Ferner ist innerhalb des Messbausteins 2 eine zentrale Datenverarbeitungseinheit 7 vorgesehen, welche sowohl an einem Sensor 3, gegebenenfalls an den Messverstärker 4, als auch an die jeweilige zweite Antenne 5 angeschlossen ist. In dieser zentralen Datenverarbeitungseinheit 7, insbesondere einem Mikrokontroller, werden die ermittelten und gegebenenfalls digitalisierten Messdaten aufbereitet, gespeichert sowie gegebenenfalls vorbereitende Analysen durchgeführt. Die zentrale Datenverarbeitungseinheit 7 ist an die zweite Antenne 5 angeschlossen, wobei zwischen die zentrale Datenverarbeitungseinheit 7 und die zweite Antenne 5 eine Kommunikationskontrolleinheit zwischengeschaltet ist, welche die Kommunikation zwischen der zentralen Datenverarbeitungseinheit 7 und einem an die Übertragungseinheit 1 angeschlossenen Rechnerknoten ermöglicht. Eine derartige Kommunikationskontrolleinheit kann auch als integrierter Bestandteil der zentralen Datenverarbeitungseinheit 7 vorliegen.

Ferner ist an der zweiten Antenne 5 eine Empfangseinheit 6 angeschlossen, welche die elektromagnetische Energie des an der zweiten Antenne 5 anliegenden Signals transformiert, zwischenspeichert und in Form eines an ihrem Ausgang anliegenden Spannungspegels zur Verfügung stellt. Diese Empfangseinheit 6 ist an einen Pufferspeicher für elektrische Energie angeschlossen, welche es erlaubt, den Betrieb der zentralen Datenverarbeitungseinheit 7, der Sensoren 3 und der Messverstärker 4 auch dann weiterzuführen, wenn zu einem gegebenen Zeitpunkt keine Energie von der Übertragungseinheit 1 an den jeweiligen Messbaustein 2 übertragen wird. Somit besteht die Möglichkeit, dass eine Messung auch dann fortgeführt werden kann, wenn keine unmittelbare Energieübertragung von der ersten Antenne 8 zur zweiten Antenne 5 erfolgt. Die gespeicherte Energie wird am Ausgang der Empfangseinheit 6 in Form eines Spannungspegels zur Verfügung gestellt, wobei die gespeicherte Energie zur Stromversorgung an die Sensoren, den Messverstärker und die zentrale Datenverarbeitungseinheit 7 abgegeben wird. Die Leitungen zur Spannungsversorgung der zentralen Datenverarbeitungseinheit, der Messverstärker sowie der Sensoren sind in Fig. 1 nicht dargestellt.

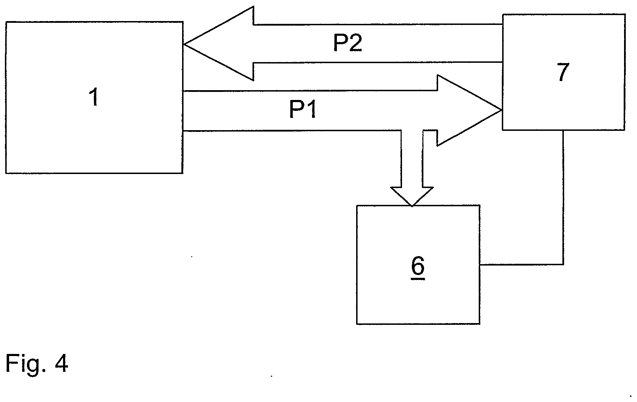

In Fig. 4 sind die Energie- und Datenübertragung zwischen der Übertragungseinheit 1 und einem Messbaustein 2 dargestellt. Von der Übertragungseinheit 1 wird elektromagnetische Energie P1 an den Messbaustein 2 übermittelt. Abgesehen von Streuverlusten wird die von der Übertragungseinheit 1 übermittelte elektromagnetische Energie P1 im Messbaustein von der zweiten Antenne 5 aufgenommene und die aufgenommene Energie zwischen der Empfangseinheit 6 und der Datenverarbeitungseinheit 7 aufgeteilt. Eine Speicherung der übermittelten Energie findet in der mit der Empfangseinheit 6 verbundenen Speichereinheit 11 statt. Diese Speichereinheit 11 umfasst einen Spannungsregler, welcher eine Versorgung des Messbausteins 2 mit konstanter Spannung ermöglicht. Die Datenverarbeitungseinheit 7 bzw. der der Datenverarbeitungseinheit 7 vorgeschaltete Kommunikationskontroller ermittelt aus den elektromagnetischen Datensignalen Steuerinformationen, welche für die Steuerung des Messbausteins 2 vorgesehen sind. Mittels dieses Signals können der Messbaustein 2 bzw. die zentrale Datenverarbeitungseinheit 7 von der Übertragungseinheit 1 angesteuert werden. Der Überschuss der Energie P1 wird an die Empfangseinheit 6 geleitet, welche die im Signal gespeicherte Energie ebenfalls in elektrische Energie umwandelt und in Form eines an ihrem Ausgang anliegenden Spannungspegels zur Verfügung stellt. Für die Messung mittels der Sensoren 3 sowie die Übermittelung von Daten von der zweiten Antenne 5 an die erste Antenne 8 steht somit jene Energie zur Verfügung, welche in der Empfangseinheit 6 gespeichert ist. Eine derartige Konfiguration des Messbausteins 2, welche ohne ständige Energiequelle auskommt, wird im folgenden als passiv bezeichnet. Nur einer der beiden Kommunikationsteilnehmer benötigt zur Aufrechterhaltung der Kommunikation eine externe Energiequelle, während der jeweils andere Kommunikationsteilnehmer, nämlich der Messbaustein 2, seine Energie aus dem Datensignal seines Kommunikationspartners ermittelt.

Zur Durchführung des erfindungsgemäßen Verfahrens, wird folgendermaßen vorgegangen: Die Übertragungseinheit 1 wird aktiv gestellt und sendet ein elektromagnetisches Signal an den ihr zugeordneten Messbaustein 2. Hierbei wird Energie in Form eines elektromagnetischen Signals von der Übertragungseinheit 1 über die erste Antenne 8 und die zweite Antenne 5 an die Empfangseinheit 6 übertragen, wodurch der am Ausgang der Empfangseinheit 6 anliegende Spannungspegel gegen einen Maximalwert strebt. Ist dieser Maximalwert erreicht, so ist ausreichend Energie zur Durchführung eines Messvorgangs vorhanden. Die erste Antenne 8 der Übertragungseinheit 1 wird inaktiv gestellt und die Messung der Impedanz der Probe mittels der beiden Sensoren 3 wird gestartet. Beachtenswert ist hier, dass die Messung nunmehr nicht durch das von der ersten Antenne 8 abgegebene elektromagnetische Signal gestört werden kann. Die durch die Interferenz zwischen dem vom Sensor 3 ausgesandten Messsignal und dem von der ersten Antenne 8 ausgesandten Datensignal entstehende Störung kann somit vollständig unterdrückt werden. Dadurch wird auch das Rauschen, welches in besonderem Maße die von den Sensoren 3 ermittelten Messwerte betrifft, weitestgehend unterdrückt.

Eine besondere Ausführungsform des erfindungsgemäßen Verfahrens betrifft die Ablaufsteuerung. Essenziell für die Durchführung einer Messung ist, dass zu Beginn des Messvorgangs so viel Energie vorhanden ist, dass die Messung zur Gänze durchgeführt werden kann. Widrigenfalls würde nämlich während der Durchführung der Messung keine Energie mehr zur Verfügung stehen, wodurch die bereits gespeicherten Messdaten eventuell wieder verloren gehen.

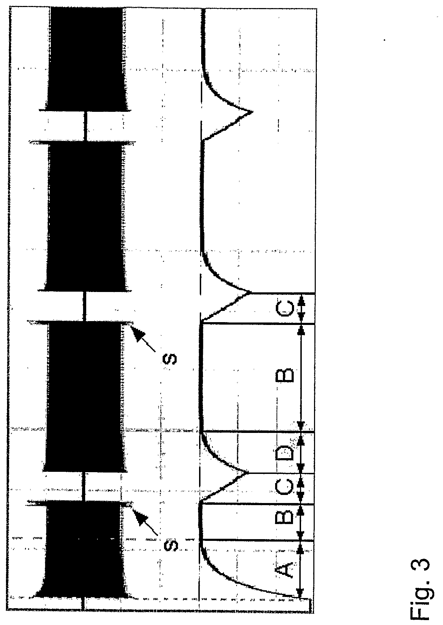

Der obere Graph der Fig. 3 stellt den Zeitverlauf des zwischen der ersten Antenne 8 und der zweiten Antenne 5 übermittelten Datensignals dar. Der untere Graph der Fig. 3 stellt die in der Empfangseinheit 6 gespeicherte elektrische Energie dar. Zu Beginn des Verfahrens ist in der Empfangseinheit 6 noch keine Energie gespeichert. Innerhalb eines Zeitbereichs A von etwa 200ms wird das von der Übertragungseinheit 1 mittels der ersten Antenne 8 und der zweiten Antenne 5 an den Messbaustein 2 übermittelte Signal zum Aufladen des Energiespeichers der Empfangseinheit 6 genutzt. Während einer Datenübertragungsphase B strebt die im Messbaustein 2 gespeicherte Energie gegen einen vorgegebenen Wert. Auch können während dieser Datenübertragungsphase B Daten zwischen dem Messbaustein 2 und der Übertragungseinheit 1 ausgetauscht werden. Am Ende der Datenübertragungsphase B sendet der Messbaustein 2 über seine zweite Antenne 5 ein Stillsetzsignal S, welches bewirkt, dass die erste Antenne 8 der Übertragungseinheit 1 inaktiv gestellt wird. Diese Inaktivsetzung erfolgt auf Veranlassung des Messbausteins 2, welcher der Übertragungseinheit 1 signalisiert, dass die an die Empfangseinheit 6 angeschlossene Speichereinheit 11 voll aufgeladen ist und im Sinne einer störungsfreien Messung weitere Signale von der Übertragungseinheit 1 zu unterbleiben haben. Die erste Antenne 8 der Übertragungseinheit 1 wird für eine vorgegebene Zeitspanne von etwa 100ms inaktiv gestellt, wobei während dieser Zeit der Messvorgang im Messbaustein 2 erfolgt. Wie im unteren Graphen dargestellt, verringert sich die in der Empfangseinheit 6 gespeicherte Energie während einer nachfolgenden Datenermittlungsphase C. Nach Ablauf einer vorgegebenen Zeit wird die erste Antenne 8 der Übertragungseinheit 1 wieder aktiv gestellt, wodurch die Empfangseinheit 6 während einer nachfolgenden Aufladephase D erneut wieder aufgeladen wird. Nach Beendigung der Aufladephase D können Daten vom Messbaustein 2 an die Übertragungseinheit 1 übertragen werden. Die im Messbaustein 2 gespeicherte Energie bleibt hierbei annähernd konstant, da trotz elektrischer Aktivität im Messbaustein 2 stetig Energie über die erste Antenne 8 an den Messbaustein 2 zugeführt wird. Nach Beendigung der Datenübertragung B gibt der Messbaustein 2 erneut einen Stillsetzimpuls S und der Messvorgang kann erneut durchgeführt werden. Typischerweise wird ein derartiges Vorgehen zehn bis zwanzig Mal wiederholt, bis alle zu ermittelten Daten mittels der Sensoren 3 aufgenommen und an die Übertragungseinheit 1 übertragen worden sind.

Eine vorteilhafte Ausführungsform der erfindungsgemäßen Anordnung ergibt sich wenn jedem Messbaustein 2 eine erste Antenne 8 zugeordnet ist und jeder ersten Antenne 8 ein eigener Antennentreiber zugeordnet ist. Nach der Reduktion des Datenaufkommens in den Antennentreibern 9 steht eine Datenleitung 10 mit geringem Datenaufkommen zur Verfügung. Diese Datenleitungen 10 können gegebenenfalls zu einem Bus zusammengeschaltet und einem gemeinsamen Rechnerknoten geführt werden.

## Patentansprüche

1. Verfahren zur Aufnahme und Übertragung von Daten zwischen einer eine erste Antenne (8) aufweisende Übertragungseinheit (1) und einem passiven Messbaustein (2), der
- einen oder mehrere Sensoren (3) zur Aufnahme von Messgrößen, insbesondere von biologischen Größen, umfasst und diese Messgrößen in elektrische Signale umwandelt, wobei den Sensoren gegebenenfalls Messverstärker (4) nachgeschaltet sind,
- wobei mit einer am Messbaustein (2) befindlichen, zweiten Antenne (5) eine bidirektionale Übermittlung von Daten, insbesondere Messdaten und Steuersignalen, zwischen dem Messbaustein (2) und der, im Nahebereich des Messbausteins (2), angeordneten Übertragungseinheit (1) mittels eines RFID- Übertragungsverfahrens erfolgt,
- wobei mit einer der zweiten Antenne (5) nachgeschalteten Empfangseinheit (6), die elektromagnetische Energie des an der zweiten Antenne (5) anliegenden Signals transformiert, zwischengespeichert wird und in Form eines an ihrem Ausgang anliegenden Spannungspegels zur Verfügung gestellt wird und die gespeicherte Energie zur Stromversorgung an die Sensoren (3), gegebenenfalls an die Messverstärker (4), abgegeben wird und,
- wobei über eine zentrale Datenverarbeitungseinheit (7) des Messbausteins (2), gespeist von der in der Empfangseinheit (6) gespeicherten Energie, zumindest die vom Messbaustein (2) umfassten Komponenten zur Weiterverarbeitung der Messsignale der Sensoren (3) und zur Kommunikation mit der Übertragungseinheit (1) gesteuert,
**dadurch gekennzeichnet, dass** für die Zeitdauer der Erfassung der Messgrößen durch die Sensoren (3) die erste Antenne (8) der Übertragungseinheit (1) bei Erhalt eines von der zweiten Antenne (5) abgegebenen Stillsetzsignals inaktiv gestellt wird, wobei die Inaktivsetzung auf Veranlassung des Messbausteins (2) erfolgt, welcher der Übertragungseinheit (1) signalisiert, dass eine an die Empfangseinheit (6) angeschlossene Speichereinheit voll aufgeladen ist.

2. Verfahren nach Anspruch 1, **dadurch gekennzeichnet, dass** das die Sensoren (3) die Impedanz der im Messbaustein (2) befindlichen biologischen Probe bei einer vorgegebenen Anzahl von Frequenzen ermitteln.

3. Verfahren nach Anspruch 1 oder 2, **dadurch gekennzeichnet, dass**
a) zur Übertragung von Energie an die Empfangseinheit (6) die erste Antenne (8) aktiv gestellt wird und elektromagnetische Energie zwischen der ersten Antenne (8) und der zweiten Antenne (5) übertragen und diese Energie im Messbaustein (2) gespeichert wird,
b) nach Übertragung einer vorgegebenen Menge elektrischer Energie ein vom Messbaustein (2) generierter Steuerimpuls an die Übertragungseinheit (1) übermittelt oder übertragen wird, wodurch die erste Antenne (8) für eine vorgegebene Zeitspanne inaktiv gesetzt wird,
c) während dieser Zeitspanne die Messung von zu bestimmenden Messgrößen erfolgt,
d) nach dem Ende der vorgegebenen Zeitspanne die Antenne (8) der Übertragungseinheit (1) wieder aktiv gesetzt wird,
e) nach Beendigung der Messung, insbesondere nach Ende der vorgegebenen Zeitspanne, die Messdaten in codierter Form vom Messbaustein an die Übertragungseinheit (1) übermittelt werden, und
f) gegebenenfalls die Schritte a) bis e) zur Durchführung einer Reihe von Messungen, gegebenenfalls von unterschiedlichen Messgrößen, für eine vorgegebene Anzahl von Wiederholungen vorgenommen werden.

4. Anordnung insbesondere zur Durchführung des Verfahrens nach einem der Ansprüche 1 bis 3, mit einer Übertragungseinheit (1) und einem Messbaustein (2), wobei die Übertragungseinheit (1) umfasst:
eine erste Antenne (8) sowie einen Antennentreiber (9) zur Ansteuerung der ersten Antenne (8) sowie zur Übermittlung von Daten mittels der ersten Antenne (8) an einen Messbaustein (2) sowie zur Aufbereitung von mittels der ersten Antenne (8) vom Messbaustein empfangenen Daten, und
wobei der , insbesondere passive, Messbaustein (2) zumindest die folgenden Komponenten umfasst:
einen oder mehrere Sensoren (3) zur Aufnahme von insbesondere physikalischen Messgrößen, insbesondere durch Bestimmung der Impedanz einer Probe, sowie deren Umwandlung in elektrische Signale, wobei den Sensoren (3) gegebenenfalls Messverstärker (4) nachgeschaltet sind,
eine zweite Antenne (5) zur bidirektionalen Übermittlung von Daten zwischen dem Messbaustein (2) und der im Nahebereich des Messbausteins (2) angeordneten Übertragungseinheit (1),
eine der zweiten Antenne (5) nachgeschaltete Empfangseinheit (6), welche die elektromagnetische Energie des an der zweiten Antenne (5) anliegenden Signals transformiert, zwischenspeichert und in Form eines an ihrem Ausgang anliegenden Spannungspegels zur Verfügung stellt und die gespeicherte Energie zur Stromversorgung an die Sensoren (3), gegebenenfalls an die Messverstärker (4), abgibt, und
eine zentrale Datenverarbeitungseinheit (7), gespeist von der in der Empfangseinheit (6) gespeicherten Energie, zur Steuerung dieser vom Messbaustein (2) umfassten Komponenten, zur Weiterverarbeitung der Messsignale der Sensoren (3) und zur Kommunikation mit der Übertragungseinheit (1),
**dadurch gekennzeichnet, dass**
der Antennentreiber (9) eine Schaltung zum zeitlich begrenzten Stillsetzen bzw. zur elektrischen Inaktivierung der ersten Antenne (8) bei Erhalt eines von der zweiten Antenne (5) abgegebenen Stillsetzsignals für die Zeitdauer der Erfassung der Messgrössen durch die Sensoren (3) aufweist und
- die Inaktivsetzung auf Veranlassung des Messbausteins (2) erfolgt, welcher der Übertragungseinheit (1) signalisiert, dass eine an die Empfangseinheit (6) angeschlossene Speichereinheit voll aufgeladen ist.

5. Anordnung nach Anspruch 4, wobei die Übertragungseinheit (1) eine Vielzahl von ersten Antennen (8) und einer Vielzahl von Messbausteinen (2) umfasst, **dadurch gekennzeichnet, dass**
a) jeder Messbaustein (2) eine eigene zweite Antenne (5) besitzt, und
b) jeder ersten Antenne (8) ein eigener bidirektionaler Antennentreiber (9) zugeschaltet ist.

## Claims

1. A method for receiving and transferring data between a transmission unit (1) having a first antenna (8) and a passive measuring module (2), which
- comprises one or more sensors (3) for receiving measurements, particularly of biological parameters, and converts these measurements into electrical signals, wherein measurement amplifiers (4) are optionally connected downstream of the sensors,
- wherein with a second antenna (5) being located on a measuring module (2) a bidirectional transmission of data, particularly measuring data and control signals, occurs between the measuring module (2) and the transmission unit (1) being arranged in close range of the measuring module (2) by means of an RFID transmission method,
- wherein with a receiving unit (6) being downstream of the second antenna (5), the electromagnetic energy of the signal abutting the second antenna (5) is transformed, buffered and provided in the form of a voltage level abutting its output and the saved energy is emitted to the sensors (3), optionally to the measurement amplifiers (4), for power supply and,
- wherein by means of a central data processing unit (7) of the measuring module (2), supplied by the energy saved in the receiving unit (6), at least the components comprising the measuring module (2) for further processing of the measuring signals of the sensors (3) and for communication with the transmission unit (1) are controlled, **characterised in that**
for the duration of the recording of the measurements by the sensors (3) the first antenna (8) of the transmission unit (1) is made inactive on receipt of a shutdown signal emitted by the second antenna (5),
wherein
- the setting to inactive occurs at the instigation of the measuring module (2), which signalises to the transmission unit (1) that a memory unit connected to the receiving unit (6) is fully charged.

2. The method according to claim 1, **characterised in that** the sensors (3) determine the impedance of the biological sample located in the measuring module (2) for a predefined number of frequencies.

3. The method according to claim 1 or 2, **characterised in that**
a) to transmit energy to the receiving unit (6) the first antenna (8) is set to active and electromagnetic energy is transmitted between the first antenna (8) and the second antenna (5) and this energy is saved in the measuring module (2),
b) after transmission of a predefined amount of electrical energy a control impulse generated by the measuring module (2) is transmitted or communicated to the transmission unit (1), whereby the first antenna (8) is made inactive for a predefined period of time,
c) during this period of time the measuring of the definable measurements occurs,
d) after the end of this predefined period of time the antenna (8) of the transmission unit (1) is set back to active,
e) after finishing the measuring, particularly at the end of the predefined period of time, the measuring data is transmitted in coded form from the measuring module to the transmission unit (1), and
f) where necessary steps a) to e) to implement a series of measurings, optionally for different measurements, are carried out for a predefined number of repeats.

4. An arrangement, particularly for executing the method according to one of claims 1 to 3, having a transmission unit (1) and a measuring module (2), wherein the transmission unit (1) comprises:
a first antenna (8) and an antenna driver (9) for controlling the first antenna (8) and for transmitting data by means of the first antenna (8) to a measuring module (2) and for preparing data received by the measuring module by means of the first antenna (8), and
wherein the, particularly passive, measuring module (2) comprises at least the following components:
one or more sensors (3) for receiving particularly physical measurements, particularly by defining the impedance of a sample, and their conversion into electrical signals, wherein measurement amplifiers (4) are optionally connected downstream of the sensors (3),
a second antenna (5) for bidirectional transmission of data between the measuring module (2) and the transmission unit (1) being arranged in close range of the measuring module (2),
a receiving unit (6) downstream of the second antenna (5), which transforms, buffers and provides in the form of a voltage level abutting the voltage level the electromagnetic energy of the signal abutting the second antenna (5) and emits the saved energy is emitted for power supply to the sensors (3), optionally to the measurement amplifiers (4), and
a central data processing unit (7), supplied by the energy saved in the receiving unit (6), for controlling these components comprising the measuring module (2), for further processing of the measuring signals of the sensors (3) and for communication with the transmission unit (1),
**characterised in that**
the antenna driver (9) has a switch for temporary shutdown or for electrical inactivation of the first antenna (8) upon receipt of a shutdown signal emitted by the second antenna (5) for the period of recording the measurements by the sensors (3) and
- the setting to inactive occurs at the instigation of the measuring module (2), which signalises to the transmission unit (1) that a memory unit connected to the receiving unit (6) is fully charged.

5. The arrangement according to claim 4, wherein the transmission unit (1) comprises a number of first antennae (8) and a number of measuring modules (2), **characterised in that**
a) each measuring module (2) has its own second antenna (5), and
b) each first antenna (8) is connected to its own bidirectional antenna driver (9).

## Revendications

1. Procédé de capture et transfert de données entre une unité de transfert (1) présentant une première antenne (8) et un module de mesure passif (2), qui
- comprend un ou plusieurs capteurs (3) pour la capture de valeurs de mesure, en particulier de valeurs biologiques et qui convertit ces valeurs de mesure en signaux électriques, dans lequel des amplificateurs de mesure (4) sont connectés en aval des capteurs,
- dans lequel, par le biais d'une seconde antenne (5) se trouvant sur le module de mesure (2), une transmission de données, en particulier de données de mesure et de signaux de commande, est effectuée entre le module de mesure (2) et l'unité de transfert (1) agencée à proximité du module de mesure (2), par l'intermédiaire d'un procédé de transfert RFID,
- dans lequel, par le biais d'une unité de réception (6) connectée en aval de la seconde antenne (5), l'énergie électromagnétique du signal présent sur la seconde antenne (5) est transformée, mise en mémoire tampon et mise à disposition sous la forme d'un niveau de tension présent à sa sortie et l'énergie stockée est fournie pour l'alimentation en courant des capteurs (3), le cas échéant des amplificateurs de mesure (4) et
- dans lequel, par le biais d'une unité de traitement de données centrale (7) du module de mesure (2), alimentée par l'énergie stockée dans l'unité de réception (6), au moins les composants compris dans le module de mesure (2) sont commandés pour le traitement ultérieur des signaux de mesure des capteurs (3) et pour la communication avec l'unité de transfert (1), **caractérisé en ce que**
pendant la durée de collecte des valeurs de mesure par les capteurs (3), la première antenne (8) de l'unité de transfert (1) est désactivée lors de la réception d'un signal d'arrêt fourni par la seconde antenne (5),
dans lequel
- la désactivation est effectuée à l'initiative du module de mesure (2), qui signale à l'unité de transfert (1) qu'une unité de stockage raccordée à l'unité de réception (6) est totalement chargée.

2. Procédé selon la revendication 1, **caractérisé en ce que** les capteurs (3) déterminent l'impédance de la sonde biologique se trouvant dans le module de mesure (2) à un nombre prédéfini de fréquences.

3. Procédé selon la revendication 1 ou 2, **caractérisé en ce que**
a) pour le transfert d'énergie à l'unité de réception (6), la première antenne (8) est activée et l'énergie électromagnétique est transférée entre la première antenne (8) et la seconde antenne (5) et cette énergie est stockée dans le module de mesure (2),
b) après le transfert d'une quantité prédéfinie d'énergie électrique, une impulsion de commande générée par le module de mesure (2) est transmise ou transférée à l'unité de transfert (1), moyennant quoi la première antenne (8) est désactivée pendant une période prédéfinie,
c) pendant cette période, la mesure des valeurs de mesure à déterminer est effectuée,
d) après la fin de la période prédéfinie, l'antenne (8) de l'unité de transfert (1) est de nouveau activée,
e) après achèvement de la mesure, en particulier après la fin de la période prédéfinie, les données de mesure sont transmises sous forme codée du module de mesure à l'unité de transfert (1) et
f) le cas échéant les étapes a) à e) sont effectuées un nombre prédéfini de fois pour la réalisation d'une série de mesures, le cas échéant de valeurs de mesure différentes.

4. Agencement en particulier pour la réalisation du procédé selon une des revendications 1 à 3, comprenant une unité de transfert (1) et un module de mesure (2), dans lequel l'unité de transfert (1) comprend :
une première antenne (8) ainsi qu'un pilote d'antenne (9) pour la commande de la première antenne (8) ainsi que pour la transmission de données au moyen de la première antenne (8) à un module de mesure (2) ainsi que pour la préparation de données reçues par le module de mesure au moyen de la première antenne (8) et
dans lequel le module de mesure (2), notamment passif, comprend les composants suivants :
un ou plusieurs capteurs (3) pour la capture de valeurs de mesure notamment physiques, en particulier par détermination de l'impédance d'une sonde, ainsi que pour leur conversion en signaux électriques, dans lequel des amplificateurs de mesure (4) sont le cas échéant connectés en aval des capteurs (3),
une seconde antenne (5) pour la transmission bidirectionnelle de données entre le module de mesure (2) et l'unité de transfert (1) disposée à proximité du module de mesure (2),
une unité de réception (6) connectée en aval de la seconde antenne (5), qui transforme l'énergie électromagnétique du signal présent sur la seconde antenne (5), la met en mémoire tampon et la met à disposition sous la forme d'un niveau de tension présent à sa sortie et qui fournit l'énergie stockée pour l'alimentation en courant des capteurs (3), le cas échéant des amplificateurs de mesure (4) et
une unité de traitement de données centrale (7), alimentée par l'énergie stockée dans l'unité de réception (6), pour la commande de ces composants compris dans le module de mesure (2), pour le traitement ultérieur des signaux de mesure des capteurs (3) et pour la communication avec l'unité de transfert (1),
**caractérisé en ce que**
le pilote d'antenne (9) présente un circuit pour l'arrêt limité dans le temps ou pour la désactivation électrique de la première antenne (8) lors de la réception d'un signal d'arrêt fourni par la seconde antenne (5) pendant la durée de détermination des valeurs de mesure par les capteurs (3) et
- la désactivation est effectuée à l'initiative du module de mesure (2), qui signale à l'unité de transfert (1) qu'une unité de stockage raccordée à l'unité de réception (6) est totalement chargée.

5. Agencement selon la revendication 4, dans lequel l'unité de transfert (1) comprend une pluralité de premières antennes (8) et une pluralité de modules de mesure (2), **caractérisé en ce que**
a) chaque module de mesure (2) possède une seconde antenne propre (5) et
b) un pilote d'antenne bidirectionnel propre (9) est connecté à chaque première antenne (8).
